# EUROPÄISCHE PATENTSCHRIFT

(11) **EP 3 744 370 B1**
(45) Veröffentlichungstag und Bekanntmachung des Hinweises auf die Patenterteilung: **02.04.2025**
(21) Anmeldenummer: 20179130.8
(22) Anmeldetag: 17.07.2012
(51) Int. Cl.: A61M 5/20, A61M 5/178, A61M 5/32

(54) **SICHERHEITSEINRICHTUNG FÜR INJEKTIONSVORRICHTUNGEN**
SAFETY ARRANGEMENT FOR INJECTION DEVICES
SYSTÈME DE SÉCURITÉ POUR DISPOSITIFS D'INJECTION

(30) Priorität: 04.08.2011 CH 12922011
(43) Veröffentlichungstag der Anmeldung: 02.12.2020
(62) Teilanmeldung aus: 17203338.3
(73) Patentinhaber: Ypsomed AG, 3401 Burgdorf (CH)
(72) Erfinder: Tschirren, Markus, 3400 Burgdorf (CH); Hirschel, Jürg, 3007 Bern (CH); Moser, Ulrich, 3412 Heimiswil (CH); Kaufmann, Nadine, 3400 Burgdorf (CH)
(74) Vertreter: Meier Obertüfer, Jürg

(56) Entgegenhaltungen:
- EP-A1- 1 932 558
- WO-A1-2006/057604
- WO-A1-2007/083115
- WO-A1-2011/005177
- WO-A1-2011/123024
- WO-A2-2009/040672
- US-A1- 2005 027 255
- US-A1- 2009 254 035

## Beschreibung

Die Erfindung betrifft eine Injektionsvorrichtung, die einen Mechanismus zum automatischen Ausschütten eines flüssigen Produkts oder Medikaments aufweist. Als flüssige Medikamente werden im Sinne der Erfindung nicht nur Flüssigkeiten im engeren Sinn angesehen, sondern auch pasten- und gelartige Medikamente, solange sich derartige Medikamente nur vergleichbar einer Flüssigkeit fördern lassen. Im Speziellen betrifft die Erfindung eine Sicherheitseinrichtung für eine Nadelschutzeinrichtung
Aus der EP1932558 und der WO2009/040672 sind Injektionsvorrichtungen bekannt, die an ihrem distalen Ende eine Nadelschutzeinrichtung und proximal von der Nadelschutzeinrichtung eine drehbare Drehhülse aufweisen. Die Drehhülse ist aus einer ersten Position in eine zweite Position drehbar und weist schräge Flächen auf, welche als Getriebemittel dienen. Die Nadelschutzeinrichtung weist Abragungen auf, die je nach Position der Nadelschutzeinrichtung die Drehhülse mittels der schrägen Fläche drehen. Befindet sich die Nadelschutzeinrichtung in ihrer distalen Position, ist die Drehhülse in ihrer ersten Position und sperrt die Bewegung der Antriebseinrichtung. Wird die Nadelschutzeinrichtung Richtung proximal zurückgeschoben, kommt die Abragung an der Nadelschutzeinrichtung mit den schrägen Fläche an der Drehhülse in Kontakt und dreht die Drehhülse von der ersten Position in die zweite Position, wodurch die Antriebseinrichtung für eine distale Bewegung in Ausschüttrichtung freigegeben wird und zugleich die Drehhülse eine Sperrposition für die Nadelschutzeinrichtung einnimmt, wenn diese sich nach der Injektion in ihrer Nadelschutzposition befindet. Damit die Drehhülse von ihrer ersten in ihre zweite Position gedreht werden kann, muss der Anwender zusätzliche Kraft aufbringen um beim Zurückschieben der Nadelschutzeinrichtung sowohl die Nadelschutzfeder zu spannen, als auch die entstehenden Haft - und Gleitreibungskräfte zwischen der Abragung und der schrägen Fläche bei der Drehbewegung der Drehhülse zu überwinden. Weitere Injektionsvorrichtungen des Standes der Technik sind bekannt aus: WO 2006/057604 A1, US 2009/254035 A1, WO 2009/040672 A2, EP 1 932 558 A1, WO 2011/123024 A1, US 2005/027255 A1, WO 2011/005177 A1 sowie WO 2007/083115 A1.

Es ist daher eine Aufgabe der Erfindung, eine Injektionsvorrichtung bereitzustellen, bei welcher der Anwender vereinfacht eine Injektion auslösen und zugleich eine Sicherheitseinrichtung für eine Nadelschutzeinrichtung aktivieren kann.

Die Aufgabe wird gelöst durch die Vorrichtung gemäss Anspruch 1. Vorteilhafte Weiterentwicklungen ergeben sich aus den abhängigen Ansprüchen.

Die Erfindung geht aus von der eingangs genannten Injektionsvorrichtung, die einen Mechanismus zum automatischen Ausschütten eines Produkts aufweist. Die Injektionsvorrichtung umfasst bevorzugt ein hülsenförmiges oder zylindrisches Gehäuse. Die Injektionsvorrichtung umfasst ferner eine Nadelschutzeinrichtung am distalen Ende der Injektionsvorrichtung oder des Gehäuses, d. h. an dem Ende, bei dem sich auch die Injektionsnadel befindet. Die Nadelschutzeinrichtung ist relativ zu dem Gehäuse verschiebbar und bevorzugt drehfest in Bezug auf das Gehäuse. Z.B. kann die Nadelschutzeinrichtung an oder in dem Gehäuse längsgeführt sein. Die Bewegbarkeit der Nadelschutzeinrichtung ist, je nach Betriebszustand der Injektionsvorrichtung eingeschränkt oder einschränkbar, insbesondere blockierbar. Die Nadelschutzeinrichtung kann aus ihrer distalen Ausgangsposition in eine proximale Position in das Gehäuse verschiebbar sein.

In der distalen Ausgangsposition nimmt die Nadelschutzeinrichtung bevorzugt eine distale Position ein, in der sie die Nadelspitze seitlich umgibt. Insbesondere befindet sich dabei die Nadelspitze proximal des distalen Endes der Nadelschutzeinrichtung. Die Nadelschutzeinrichtung ist vorzugsweise hülsenförmig. Die Nadelschutzeinrichtung kann ausserhalb oder vorzugsweise innerhalb des Gehäuses angeordnet sein. In der distalen Ausgangsposition steht zumindest das distale Ende der Nadelschutzeinrichtung bevorzugt distal über das distale Ende des Gehäuses. In einer proximalen Position ist die Nadelschutzeinrichtung in Bezug auf das Gehäuse zurückgeschoben. Wobei die Nadelschutzeinrichtung mit einem geringeren Mass über das distale Ende des Gehäuses steht als in der distalen Ausgangsposition der Nadelschutzeinrichtung oder aber mit dem Gehäuse bündig ist.

Bevorzugt ist die Nadelschutzeinrichtung durch Andrücken ihres distalen Endes an eine Injektionsstelle in die zurückgeschobene bzw. proximale Position bewegbar, wodurch die Nadel aus der Nadelschutzeinrichtung hervortritt und in das Körpergewebe einstechen kann. Durch die Bewegung der Nadelschutzeinrichtung in die proximale Position kann die relativ zu dem Gehäuse feststehende Nadel aus der Nadelschutzeinrichtung hervortreten und/ oder, es kann ein Vortriebsmechanismus, z.B. eine Vortriebsfeder enthaltend, ausgelöst werden, der zumindest die Nadel relativ zum Gehäuse in distale Richtung verschiebt, wodurch die Nadel aus der Nadelschutzeinrichtung hervortritt.

Bevorzugt ist die Nadelschutzeinrichtung am Ende der Bewegung aus der distalen Ausgangsposition in die zurückgeschobene Position und in distaler Richtung zurück in eine Nadelschutzposition an einer weiteren Bewegung in die distale und proximale Richtung insbesondere gesperrt. Vorteilhaft ist die Nadelschutzeinrichtung nach der Verwendung der Vorrichtung axial fest insbesondere mit und/ oder relativ zu dem Gehäuse verriegelt. Dadurch kann die Nadelschutzeinrichtung nicht mehr in das Gehäuse zurückgeschoben werden, wodurch die Nadel nicht mehr aus dem distalen Ende der Nadelschutzeinrichtung hervortreten kann und wodurch die von der Nadel ausgehende Verletzungsgefahr verringert wird.

In bevorzugten Ausführungen kann die Injektionsvorrichtung ferner einen Drehknopf aufweisen, der aus einer ersten nicht gedrehten Sperr-Position bewegbar, insbesondere drehbar ist. Der Drehknopf ist vorzugsweise an dem proximalen Ende der Injektionsvorrichtung oder des Gehäuses angeordnet, insbesondere ist der Drehknopf in Bezug auf das Gehäuse um dessen Längsachse drehbar und axial fest.

In der Sperr-Position des Drehknopfs ist die Nadelschutzeinrichtung in ihrer Bewegbarkeit in die proximale Position blockiert. In der Freigabe-Position des Drehknopfs ist die Nadelschutzeinrichtung hingegen in eine proximale Position oder eine Nadelschutzposition verschiebbar. Dadurch kann in der Sperr-Position die Nadel nicht über das distale Ende der Nadelschutzeinrichtung hervortreten und in der Freigabe-Position die Nadel über das distale Ende der Nadelschutzeinrichtung hervorstehen oder hervortreten.

Die Nadelschutzeinrichtung kann in bevorzugten Ausführungen eine Sperrrippe, mit der die Nadelschutzeinrichtung gegen Zurückschieben aus der distalen Ausgangsposition in die proximale Position blockierbar ist, und ein federnd angeordnetes Schnappelement, mit dem die Nadelschutzeinrichtung gegen Zurückschieben aus der Nadelschutzposition in die proximale Position blockierbar ist aufweisen.

Befindet sich die Nadelschutzeinrichtung in ihrer distalen Ausgangsposition ist vorzugsweise die Sperrrippe in einer Sperrposition mit einem in proximaler Richtung wirkenden Anschlag. Der in proximale Richtung wirkende Anschlag kann auch als Anfangsanschlag bezeichnet werden, da er eine Bewegung der Nadelschutzeinrichtung aus der distalen Anfangsposition in proximale Richtung sperrt. Bei dem Versuch die Nadelschutzeinrichtung in proximale Richtung zu verschieben, wird die Sperrrippe gegen den Anfangsanschlag gedrückt. Die Bewegung der Nadelschutzeinrichtung aus der distalen Ausgangsposition in proximale Richtung kann entsperrt werden. Mittels der Drehung des Drehknopfs von der Sperr-Position in die Freigabe-Position können die Sperrrippe und der Anfangsanschlag aus dem Anschlag bewegt werden. Beispielsweise kann der Anfangsanschlag relativ zu der Sperrrippe gedreht werden. Dadurch, dass die Sperrrippe und der Anfangsanschlag keinen Anschlag mehr bilden, ist die Nadelschutzeinrichtung in die proximale Position bewegbar.

Bevorzugt ist das Schnappelement, das bei einer Bewegung der Nadelschutzeinrichtung aus ihrer proximalen Position in distale Richtung, d. h. bei der Bewegung in die Nadelschutzposition, von einer Führung, insbesondere einer Rampe relativ zur Nadelschutzeinrichtung federnd radial auslenkbar, wobei das Schnappelement in der Nadelschutzposition der Nadelschutzeinrichtung aus der ausgelenkten Position zurückfedern kann. Während der Bewegung der Nadelschutzeinrichtung in ihre distale Nadelschutzposition kann das Schnappelement an der Rampe entlang gleiten und wird quer zur Längsachse gespannt. In der distalen Nadelschutzposition der Nadelschutzeinrichtung, federt das Schnappelement in Radialrichtung zu der Längsachse der Injektionsvorrichtung hin, oder von jener weg, d. h. in entgegengesetzte Richtung, in die es ausgelenkt und gespannt wurde. Dabei gelangt das Schnappelement an einen in proximaler Richtung wirkenden Anschlag, der auch als Endanschlag bezeichnet werden kann, da er die Nadelschutzeinrichtung an einer Bewegung aus ihrer distalen Nadelschutzposition in eine proximale Position insbesondere sperrt.

In alternativen bevorzugten Ausführungen kann über eine Drehung einer Drehhülse von einer ersten in eine zweite Position ein Schnappelement und oder eine Sperrrippe an einer Steuerhülse über eine Rampe quer zur Längsachse ausgelenkt werden, wodurch dann ein Endanschlag für die Nadelschutzeinrichtung in der Nadelschutzposition gebildet wird, welcher ein Zurückschieben der Nadelschutzeinrichtung in eine proximale Position insbesondere sperrt.

Die Drehhülse der Injektionsvorrichtung ist ferner mit wenigstens einem Anfangsanschlag für die Nadelschutzeinrichtung versehen. Die Drehhülse kann während der Bewegung des Drehknopfs von der Sperr-Position in die Freigabe-Position von einer nicht aktivierten Position in die erste Position gedreht werden. Dabei wird der Anfangsanschlag von der Sperrrippe aus dem Anschlag weg bewegt und die Nadelschutzeinrichtung kann in die proximale Position bewegt werden. Ferner weist die Drehhülse zumindest ein Profil auf welches als Getriebefläche ausgebildet ist.

Bevorzugt ist die Nadelschutzeinrichtung eine Auslöseeinrichtung, die zum Auslösen der Produktausschüttung in das Gehäuse oder die proximale Position verschiebbar ist. Hierdurch kann die Antriebseinrichtung und/ oder das Antriebsmittel zum Ausschütten des Wirkstoffs freigegeben werden.

Die Erfindung wurde anhand mehrerer Ausführungsformen beschrieben. Im Folgenden werden weitere bevorzugte Ausführungen anhand von Figuren beschrieben. Die dabei offenbarten Merkmale bilden den Gegenstand der Erfindung einzeln und in jeder Merkmalskombination vorteilhaft weiter. Es zeigen:
- Figur 1: Eine Explosionsdarstellung einer ersten Ausführungsform einer Injektionsvorrichtung,
- Figuren 2 und 3: Schnittdarstellungen der Injektionsvorrichtung im Anfangszustand mit aufgesetzter Kappe, wobei Figur 3 eine gegenüber Figur 2 um 90° um die Längsachse gedrehte Ansicht ist,
- Figur 4: Detailansicht aus Figur 2 und 3 wobei sich die Nadelschutzeinrichtung mit der Drehhülse im Sperreingriff befindet,
- Figur 5: Schnittdarstellung der Injektionsvorrichtung, wobei der Drehknopf in einer Sperr-Position ist,
- Figur 6: Schnittdarstellung der Injektionsvorrichtung wobei der Drehknopf um die Längsachse gedreht wurde und sich in der Freigabe-Position befindet,
- Figur 7: Detailansicht einer zweiten Ausführungsform einer Injektionsvorrichtung vor Vollendung der Montage,
- Figur 8: Detailansicht der Injektionsvorrichtung wobei die Drehhülse in ihre erste Position gedreht wurde und die Antriebseinrichtung in Richtung Ausschüttposition gesperrt ist,
- Figur 9: Schnittdarstellung der Injektionsvorrichtung, wobei die Kappe abgezogen wurde
- Figuren 10 und 11: Schnittansichten der Injektionsvorrichtung, wobei die Antriebseinrichtung in Richtung Ausschüttposition entsperrt wurde,
- Figur 12: Schnittansicht der Injektionsvorrichtung, wobei die Nadelschutzeinrichtung sich in der proximalen Position befindet, und die Drehhülse um die Längsachse in ihre zweite Position gedreht wurde
- Figuren 13 und 14: Schnittansichten der Injektionsvorrichtung, wobei sich Antriebseinrichtung in der Ausschüttposition befindet und das Klickelement freigegeben wurde,
- Figuren 15 bis 16: Schnittansichten der Injektionsvorrichtung im Endzustand, wobei die Nadelschutzeinrichtung sich in der Nadelschutzposition befindet und die Drehhülse die Bewegung der Nadelschutzeinrichtung sperrt,
- Figur 17: Eine Explosionsdarstellung einer dritten Ausführungsform einer Injektionsvorrichtung,
- Figuren 18 und 19: Schnittdarstellungen der Injektionsvorrichtung im Anfangszustand mit aufgesetzter Kappe, wobei Figur 19 eine gegenüber Figur 18 um 90° um die Längsachse gedrehte Ansicht ist,
- Figuren 20 und 21: Schnittdarstellungen der Injektionsvorrichtung, wobei Figur 21 eine gegenüber Figur 20 um 90° um die Längsachse gedrehte Ansicht ist, wobei die Kappe abgezogen wurde, die Nadelschutzeinrichtung in ihre proximale Position bewegt wurde und die Antriebseinrichtung in Richtung Ausschüttposition entsperrt wurde, wobei die Drehung der Drehhülse um die Längsachse in ihre zweite Position freigegeben wurde,
- Figur 22: Schnittansicht der Injektionsvorrichtung, wobei die Drehhülse zum Drehen von ihrer ersten in ihre zweite Position freigegen wurde,
- Figur 23: Schnittansicht der Injektionsvorrichtung, wobei die Drehhülse um die Längsachse in ihre zweite Position gedreht wurde und die Sperre der Nadelschutzeinrichtung aktiviert wurde,
- Figur 24: Schnittansicht der Injektionsvorrichtung, wobei sich die Antriebseinrichtung in der Ausschüttposition befindet und das Klickelement freigegeben wurde,
- Figur 25: Schnittansicht der Injektionsvorrichtung im Endzustand, wobei die Nadelschutzeinrichtung sich in der Nadelschutzposition befindet und über die Drehhülse in Ihrer Bewegung gesperrt ist,
- Figur 26: Eine Explosionsdarstellung einer vierten Ausführungsform einer erfindungsgemäßen Injektionsvorrichtung
- Figuren 27 und 28: Schnittdarstellungen der Injektionsvorrichtung im Anfangszustand mit aufgesetzter Kappe, wobei Figur 28 eine gegenüber Figur 27 um 90° um die Längsachse gedrehte Ansicht ist,
- Figur 29: Schnittdarstellung der Injektionsvorrichtung, wobei die Kappe abgezogen wurde, die Nadelschutzeinrichtung in ihre proximale Position bewegt wurde und die Antriebseinrichtung in Richtung Ausschüttposition entsperrt wurde, wobei die Drehung der Drehhülse um die Längsachse in ihre zweite Position freigegeben wurde,
- Figur 30: Schnittansicht der Injektionsvorrichtung, wobei die Drehhülse zum Drehen von ihrer ersten in ihre zweite Position freigegen wurde,
- Figuren 31 und 32: Schnittansichten der Injektionsvorrichtung, wobei sich die Antriebseinrichtung in der Ausschüttposition befindet und das Klickelement freigegeben wurde,
- Figuren 33 bis 35: Schnittansichten der Injektionsvorrichtung, wobei die Nadelschutzeinrichtung sich in ihre Nadelschutzposition bewegt und die Drehhülse um die Längsachse gedreht wird, und die Sperre der Nadelschutzeinrichtung aktiviert wird,
- Figur 36: Schnittansicht der Injektionsvorrichtung, wobei die Nadelschutzeinrichtung sich in der Nadelschutzposition befindet und über die Drehhülse in Ihrer Bewegung gesperrt ist.

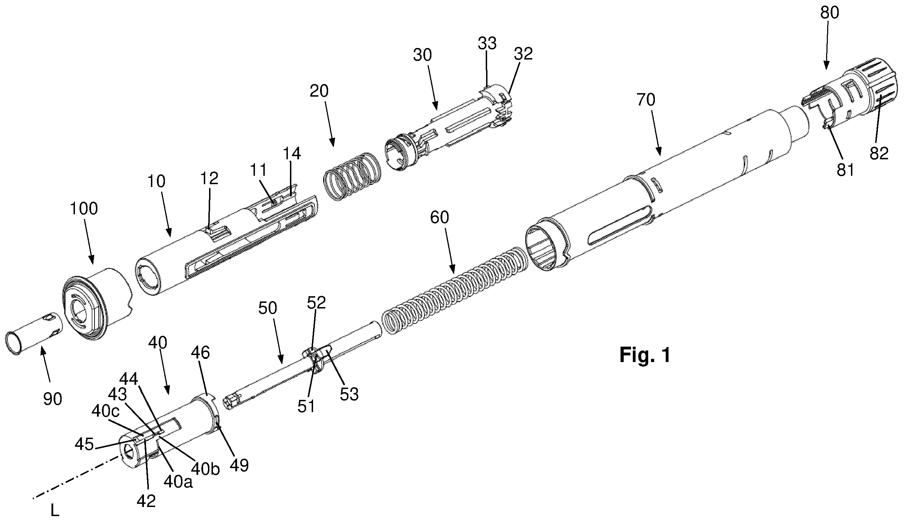

Zunächst werden mit Bezug auf Figur 1 die Einzelteile einer Injektionsvorrichtung beschrieben. Die Injektionsvorrichtung umfasst eine Nadelschutzeinrichtung 10, ein hülsenförmiges Gehäuse 70, wobei die Nadelschutzeinrichtung in dem Gehäuse 70 um die Längsachse L längsverschiebbar aufgenommen wird und gleichzeitig als Auslöseelement dient, ein Spritzenhalter 30 welcher axial fest mit dem Gehäuse 70 verbindbar ist und worin ein Wirkstoffbehälter eingesetzt werden kann, eine Antriebseinrichtung 50 welche auf den Wirkstoffbehälter 110 wirken kann, in der Gestalt einer Hülse mit einer tellerartigen Halterung 52, und ein Antriebsmittel 60 in der Gestalt einer als Druckfeder wirkenden Wendelfeder, die insbesondere die Energie für eine Ausschüttsequenz liefert, eine Drehhülse 40, die die Antriebseinrichtung 50 und das Antriebsmittel 60 bis zur Auslösung der Injektionsvorrichtung in einem gespannten Zustand hält und die axialfest mit dem Gehäuse 70 verbunden, insbesondere verschnappt ist, und die vor und nach einer Injektion die axiale Bewegung der Nadelschutzeinrichtung 10 sperren kann, eine Nadelschutzfeder 20, die die Energie für die Verschiebung der Nadelschutzeinrichtung 10 in die Nadelschutzposition oder eine zweite Position liefern kann, und ein Drehknopf 82 der die Drehhülse 40 insbesondere aus einer nicht aktivierten Position drehen kann.

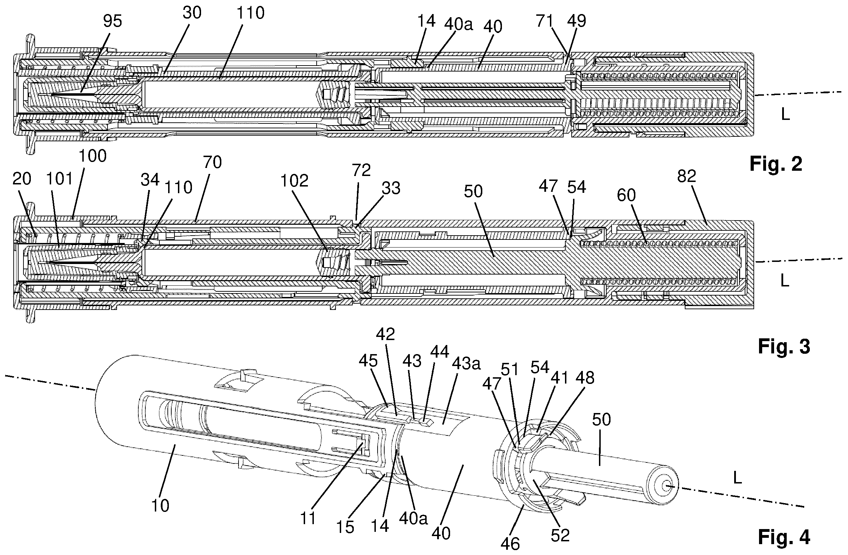

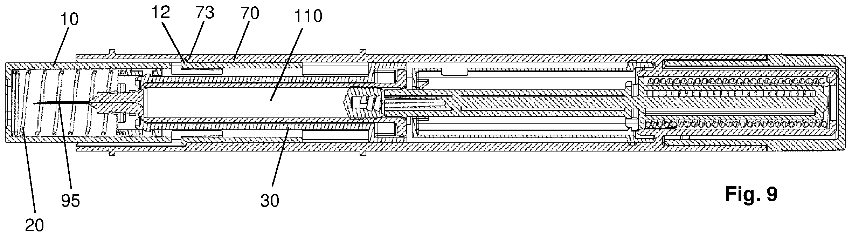

Die Figuren 2 bis 16 beschreiben eine Injektionsvorrichtung, die angepasst ist ein in einem Wirkstoffbehälter 110 enthaltenes Produkt automatisch auszuschütten. Das Einstechen erfolgt manuell, d. h. per Hand. Die Vorrichtung wird in den Figuren 2 bis 4 in einem Auslieferungszustand dargestellt. Der Drehknopf 82 befindet sich in seiner ersten nicht gedrehten Sperr-Position. Der Wirkstoffbehälter 110 ist in dem Spritzenhalter 30 angeordnet und stützt sich mit einer Schulter, die distal des Wirkstoffbehälters 110 angeordnet ist, an einer nach innen gerichteten Schulter 34 entlang des Spritzenhalters 30 ab.

Der Spritzenhalter 30 ist über die Haltemittel 33 im Gehäusedurchbruch 72 axial fest mit dem Gehäuse 70 verbunden, insbesondere verschnappt. Eine Nadel 95 ist mit dem Wirkstoffbehälter 110 verbunden. Die Nadel 95 ragt in dem Auslieferungszustand der Vorrichtung über das distale Ende des Gehäuses 70. Die Nadel 95 wird von einer Nadelschutzkappe 101 abgedeckt. Die Kappe 100 der Vorrichtung weist einen Kappeneinsatz 90 auf welcher z. B. eine Kralle aus Metall oder Kunststoff oder eine Hülse sein kann, somit ist die Kappe so ausgestaltet, dass die Nadelschutzkappe 101 von der Vorrichtung entfernt wird, wenn die Kappe 100 von der Vorrichtung durch Abziehen entfernt wird.

Der Wirkstoffbehälter 110 umfasst einen Kolben 102, der relativ zu dem Wirkstoffbehälter 110 für eine Produktausschüttung verschiebbar ist. Der Kolben 102 ist mittels einer Antriebseinrichtung 50, die proximal des Kolbens 102 angeordnet ist, verschiebbar. Zwischen Antriebseinrichtung 50 und dem Gehäuse 70 ist ein Antriebsmittel 60 angeordnet, das im Auslieferungszustand der Vorrichtung vorgespannt ist und mit dem die Antriebseinrichtung 50 in Ausschüttrichtung antreibbar ist.

Die Antriebseinrichtung 50 umfasst mindestens eine, vorzugsweise zwei Halterungsflächen 54 die in einem Eingriff mit einer Stützfläche 47 an der Drehhülse 40 verhindern, dass das Antriebsmittel 60 die in ihm gespeicherte Energie zum Vortrieb auf die Antriebseinrichtung 50 abgeben kann. Die Halterungsfläche 54 ist an einer tellerartigen Halterung 52 ausgebildet und ragt an dieser ab. Die Drehhülse 40 ist mittels Nocken 49 im Halterungsschlitz 71 relativ zum Gehäuse axial gehalten und rotativ bewegbar.

Die Vorrichtung umfasst ferner eine Nadelschutzeinrichtung 10 in der Gestalt einer Hülse, welche auch als Betätigungshülse für die Produktausschüttung dient. Die Nadelschutzeinrichtung 10 ist relativ zu dem Gehäuse 70 verdrehgesichert axial verschiebbar. Die Nadelschutzeinrichtung 10 ragt in einem Ausgangszustand distal über das distale Ende des Gehäuses 70 und über das distale Ende der Nadel 95. Zwischen der Nadelschutzeinrichtung 10 und dem Wirkstoffbehälter 110 oder dem Spritzenhalter 30 ist eine Feder 20 angeordnet. Die Feder 20 wirkt als eine Druckfeder und kann die Nadelschutzeinrichtung 10 aus einer proximalen Position in eine distale Position verschieben. Die Nadelschutzeinrichtung 10 wird mittels Anschlägen 15 gegen ein Herausfallen aus der Vorrichtung in distale Richtung über Anschläge 32 an dem Spritzenhalter gehalten.

Wie am besten aus der Figur 4 erkennbar ist, weist die Drehhülse 40 an ihrem äusseren Umfang mindestens eine insbesondere parallel zur Längsachse L verlaufende erste Führungsrippe 42 und mindestens eine zweite Führungsrippe 44 auf. Zwischen der ersten und der zweiten Führungsrippe befindet sich eine erste Nut 43, proximal von der zweiten Führungsrippe 44 befindet sich eine zweite Nut 43a. Quer insbesondere senkrecht zu der ersten Führungsrippe 42 erstreckt sich in Längsrichtung eine Rampe 45. Weiter ist auf dem Umfang der Drehhülse 40 quer zur Längsachse L mindestens ein Anfangsanschlag 40a zum Sperren der Nadelschutzeinrichtung 10 in proximale Richtung angebracht. Die Nadelschutzeinrichtung 10 weist an ihrem inneren Umfang mindestens eine Sperrrippe 14 auf.

Versetzt von der Sperrrippe 14 ist mindestens ein federnd und insbesondere nach innen gerichtetes Schnappelement 11 angebracht. Bevorzugt weist, die Sperrrippe 14 selbst ein Schnappelement 11 auf, oder ist einteilig mit einem Schnappelement 11 ausgebildet. Im Auslieferungszustand wird die Nadelschutzeinrichtung 10 mittels der Sperrrippe 14 und dem Anfangsanschlag 40a an der Drehhülse 40 gegen ein axiales Verschieben in proximale Richtung gesperrt.

Die Drehhülse 40 weist des Weiteren an ihrer Innenwand mindesten eine Stützfläche 47 auf, wodurch die Halterungsfläche 54 der tellerartigen Halterung 52 der Antriebseinrichtung 50 vor der Entsicherung der Injektionsvorrichtung aufliegen kann und das Antriebsmittel 60 welches auf die Antriebseinrichtung 50 wirkt, im gespannten Zustand gehalten wird. Radial versetzt von der Stützfläche 47 der Drehhülse 40 ist ein erstes Profil 41 angebracht, welches nach dem Entsichern der Injektionsvorrichtung mit dem zweiten Profil 51 an der Antriebseinrichtung 50 ein Getriebe bildet. Das erste Profil 41 ist als eine schräge Fläche insbesondere als ein Steg ausgebildet, welcher in der Führung 48 endet und somit eine Rampe für die Antriebseinrichtung 50 bildet. Die Drehhülse 40 ist an ihrem proximalen Ende mit mindestens einer Klaue 46 ausgebildet, welche zum Entsichern der Injektionsvorrichtung mit einer Gegenklaue 81 an dem Drehknopf 82 zusammenwirkt.

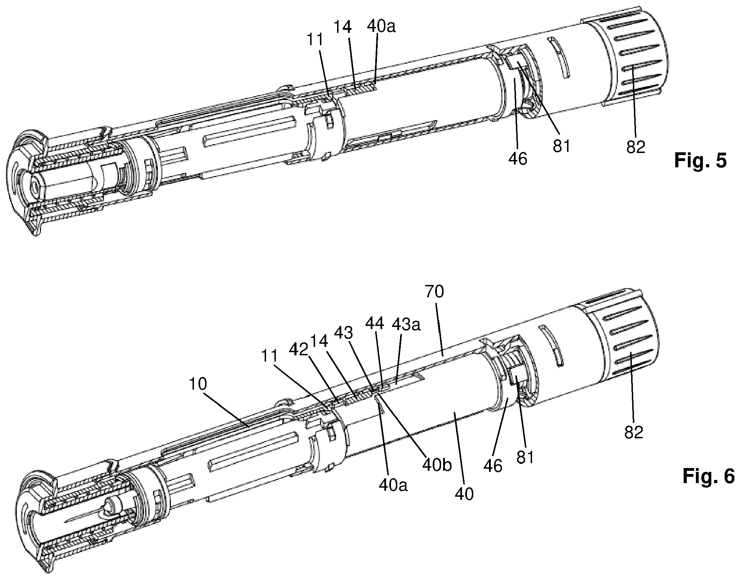

Zum Verabreichen des Produkts entsichert der Anwender die Injektionsvorrichtung, indem er den Drehknopf 82 von einer Sperr-Position (Figur 5) in eine Freigabe-Position (Figur 6) dreht. Danach kann der Anwender die Kappe 100 der Vorrichtung abnehmen. Es ist auch möglich zuerst die Kappe 100 abzunehmen und danach den Drehknopf 82 zu drehen. Über die Drehung des Drehknopfs 82 wird mittels der Gegenklaue 81 am Drehknopf 82 und der Klaue 46 an der Drehhülse 40, die Drehhülse 40 von einer nicht aktivierten Position (Figur 5) in eine erste Position gedreht (Figur 6).

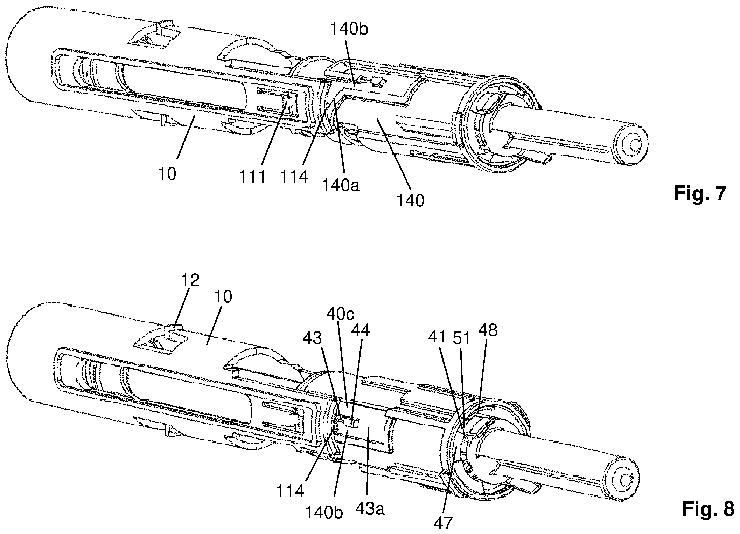

Über die Drehung der Drehhülse 40 gelangen die Sperrrippe 14 und das Schnappelement 11, an der Nadelschutzeinrichtung in die erste Führung 40b der Drehhülse 40. Wie in der Figur 7 dargestellt, kann der Anfangsanschlag 40a als eine schräge Fläche insbesondere als eine Getriebefläche 140a ausgebildet sein. Dabei erfolgt die Drehung der Drehhülse 140 aus einer nicht aktivierten Position in eine erste Position über eine axiale erste Verschiebung der Nadelschutzeinrichtung 10 in proximaler Richtung, wobei die Sperrrippe 114 über die Getriebefläche 140a die Drehhülse 140 dreht, so dass die Sperrrippe 114 und das Schnappelement 111 in die erste Führung 140b gelangen.

Die Antriebseinrichtung 50 ist mit dem Gehäuse 70 verdrehgesichert. Durch die Drehbewegung der Drehhülse 40 von der nicht aktivierten Position in die erste Position kommen das erste Profil 41 und das zweite Profil 51 zum Aufliegen und bilden das Getriebe. Die in Ausschüttrichtung wirkende Kraft des Antriebsmittels 60, wird durch das zweite Profil 51 der Antriebseinrichtung 50 auf das erste Profil 41 der Drehhülse 40 in ein Drehmoment gewandelt, welches versucht die Drehhülse 40 aus ihrer ersten Position um die Längsachse L in ihre zweite Position zu drehen. Da die Sperrrippe 14 und das Schnappelement 11 der Nadelschutzeinrichtung 10 sich in der ersten Führung 40b befinden, wird die Drehbewegung der Drehhülse 40 über die erste und zweite Führungsrippe 42, 44 der Drehhülse 40 gesperrt. Hingegen ist die Nadelschutzeinrichtung 10 in der in Figur 8 gezeigten Position nicht mehr für eine axiale Verschiebung in proximale Richtung gesperrt.

Zur Verabreichung des Produkts umgreift der Anwender das Gehäuse 70 und drückt die Injektionsvorrichtung mit dem distalen Ende der Nadelschutzeinrichtung 10 gegen die gewünschte Einstechstelle. Durch das Aufdrücken des Gehäuses 70 werden die Komponenten der Injektionsvorrichtung inklusive des Wirkstoffbehälters 110 und der Nadel 95 relativ zu der Nadelschutzeinrichtung 10 in distale Richtung verschoben, wobei die Nadel 95 in die Injektionsstelle einsticht.

Ein Anfangswiderstand soll dafür sorgen, dass die Andrückkraft des Anwenders ansteigt und die Nadel 95 mit genügend Kraft eingestochen wird. Hierzu weist die Nadelschutzeinrichtung mindestens einen flexiblen Schnapper 12 auf, welcher insbesondere radial nach aussen zur Gehäuseinnenfläche gerichtet ist. Das Gehäuse 70 weist auf der Innenseite mindestens ein Sperrmittel 73 auf. Wird bei dem Versuch die Nadelschutzeinrichtung 10 in das Gehäuse 70 zurückzuschieben der Schnapper 12 gegen das Sperrmittel 73 gedrückt, kann der Schnapper zunächst nicht den Widerstand des Sperrmittels 73 überwinden. Dieser Anfangswiderstand behindert das Zurückschieben der Nadelschutzeinrichtung 10, so dass beim Andrücken der Injektionsvorrichtung an die Injektionsstelle zunächst eine gewisse Anpresskraft zur Überwindung des Anfangswiderstands aufgewendet werden muss, die, sobald sie den Schnapper 12 aus dem Eingriff mit dem Sperrmittel 73 gedrückt hat schlagartig wegfällt, wodurch die Nadelschutzeinrichtung 10 schlagartig zurückgeschoben und die Nadel 95 schlagartig eingestochen wird.

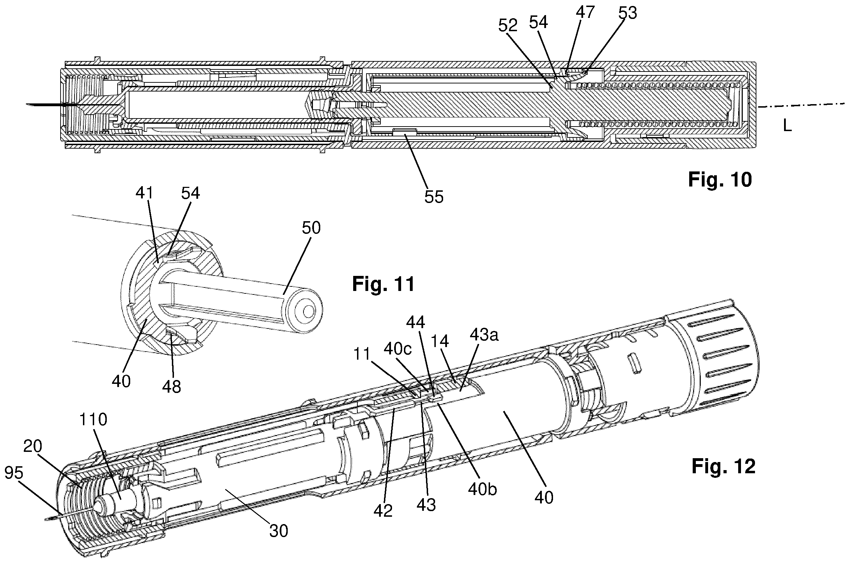

Figuren 10 bis 12: Bei der Bewegung der Nadelschutzeinrichtung 10 in die proximale Position wird das Schnappelement 11 von der ersten Führungsrippe 42 und die Sperrrippe 14 von der zweiten Führungsrippe 44 wegbewegt, so dass das Schnappelement 11 sich bei der ersten Nut 43 und die Sperrrippe 14 sich bei der zweiten Nut 43a befinden. In diesem Zustand der Nadelschutzeinrichtung 10 ist die Drehbewegung der Drehhülse 40 von der ersten Position in die zweite Position freigegeben. Durch das vom Getriebe (mittels der Profile 41 und 51) aufgebrachte Drehmoment wird die Drehhülse 40 gedreht, wobei die Stützfläche 47 der Drehhülse 40 von der Halterungsfläche 54 der Antriebseinrichtung 50 weggedreht wird und die Halterungsfläche 54 in die Führung 48 gelangt. Dabei wird die Antriebseinrichtung 50 entsperrt und kann mittels des Antriebsmittels 60 in Ausschüttrichtung getrieben werden, wodurch der Wirkstoff aus dem Wirkstoffbehälter 110 ausgeschüttet wird.

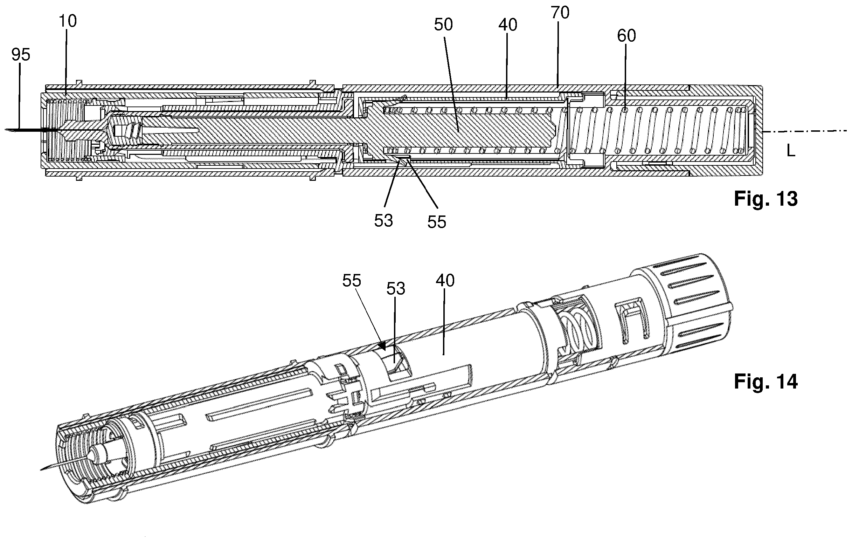

Mindestens ein Klickelement 53 an der Halterung 52 der Antriebseinrichtung 50 wird dabei an der Führung 48 der Drehhülse 40 nach innen eingelenkt. Am Ende der Ausschüttsequenz, d. h. der Bewegung der Antriebseinrichtung 50 in Ausschüttrichtung, federt das zumindest eine Klickelement 53 in die an der Drehhülse 40 gebildete Aussparung 55, wodurch ein Klickgeräusch das Ende der Ausschüttsequenz signalisiert. Dieser Zustand wird in den Figuren 13 und 14 gezeigt.

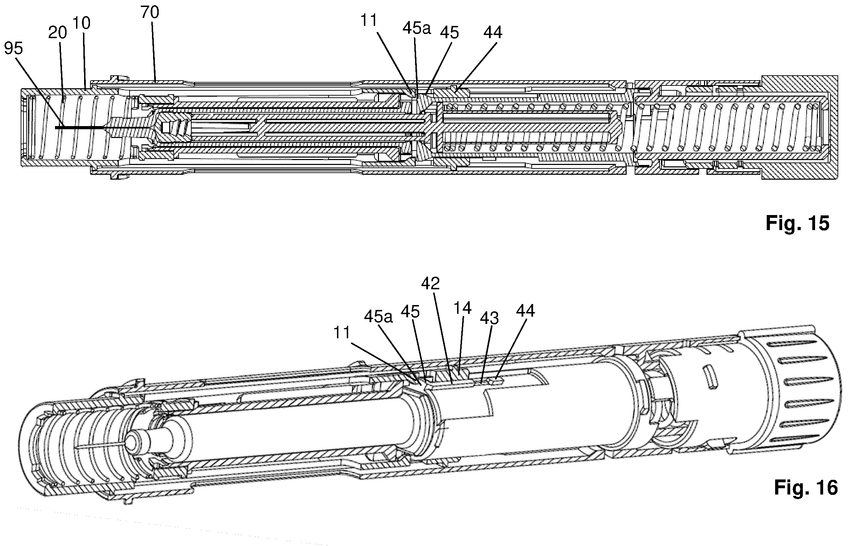

Nach dem Ende der Ausschüttsequenz und nachdem der Anwender optional 1 bis 30 Sekunden gewartet hat, bis sich das Medikament in dem Gewebe verteilt hat, zieht der Anwender die Injektionsvorrichtung von der Einstechstelle zurück, wodurch die Nadelschutzfeder 20 die Nadelschutzeinrichtung 10 relativ zu dem Gehäuse 70 in die distale Richtung so weit verschiebt, bis die Nadelschutzeinrichtung 10 über das distale Ende der Nadel 95 steht und somit ihre Nadelschutzposition einnimmt, in der ein Zugriff auf die Nadel 95 verhindert wird. Wie am besten aus den Figuren 15 und 16 ersichtlich ist, bewegen sich das Schnappelement 11 und die Sperrrippe 14 entlang der zweiten Führung 40c in distale Richtung wenn die Nadelschutzeinrichtung 10 in ihre Nadelschutzposition bewegt wird.

Während oder insbesondere am Ende der Bewegung der Nadelschutzeinrichtung 10 in die Nadelschutzposition federt das Schnappelement 11 an der Rampe 45 entlang und schnappt vor einen Endanschlag 45a ein, der distal der Rampe 45 angeordnet ist. Der Endanschlag 45a wirkt als Axialanschlag, gegen den das Schnappelement 11 gedrückt wird, wenn versucht wird die Nadelschutzeinrichtung 10 aus ihrer Nadelschutzposition in proximale Richtung zu verschieben. Somit ist die Nadelschutzeinrichtung 10 gegen ein Zurückschieben blockiert, wodurch die Verletzungsgefahr für den Anwender der Vorrichtung verringert wird.

In der Nadelschutzposition der Nadelschutzhülse 10 befindet sich die Sperrrippe 14 in der zweiten Führung 40c, in der sogenannten Rückdrehsperre 40c und wird über die erste Führungsrippe 42 seitlich eingeschlossen. Damit ist die Drehhülse 40 gegen ein Drehen um die Längsachse L in beide Drehrichtungen gesichert.

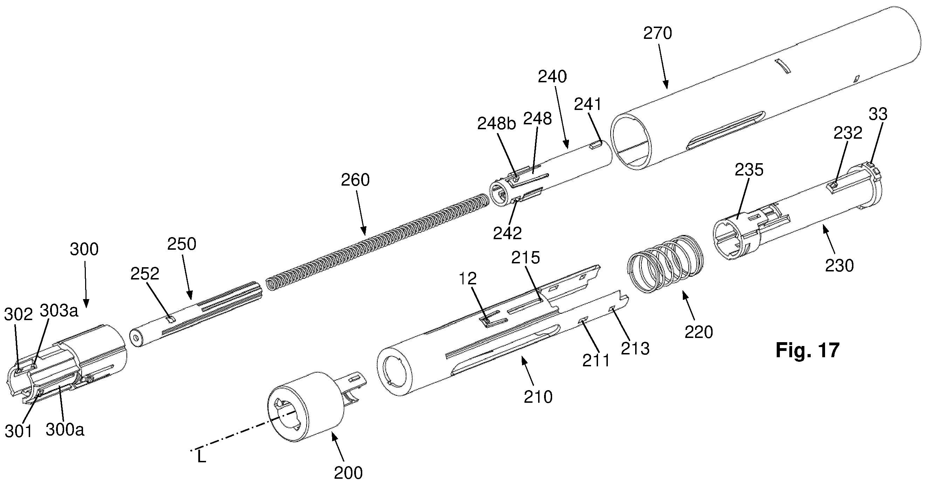

In den Figuren 17 bis 25 wird eine weitere, dritte Ausführungsform einer erfindungsgemässen Injektionsvorrichtung gezeigt.

In der Figur 17 werden die Einzelteile der Injektionsvorrichtung gezeigt. Die Injektionsvorrichtung umfasst eine Nadelschutzeinrichtung 210, ein hülsenförmiges Gehäuse 270, wobei die Nadelschutzeinrichtung 210 in dem Gehäuse 270 um die Längsachse L längsverschiebbar aufgenommen wird und gleichzeitig als Auslöseelement dient, ein Spritzenhalter 230 welcher axial fest mit dem Gehäuse 270 verbindbar ist und worin ein Wirkstoffbehälter 110 eingesetzt werden kann, ein Haltering 235, welcher distal am Spritzenhalter 230 angebracht wird um den Wirkstoffbehälter 110 im Spritzenhalter 230 zu sichern, eine Antriebseinrichtung 250 welches auf den Wirkstoffbehälter 110 wirken kann, in der Gestalt einer Hülse welche an ihrer Aussenwand mindestens eine Halterungsfläche 252 aufweist, und ein Antriebsmittel 260 in der Gestalt einer als Druckfeder wirkenden Wendelfeder, die insbesondere die Energie für eine Ausschüttsequenz liefert, eine Drehhülse 240, die die Antriebseinrichtung 250 und das Antriebsmittel 260 bis zur Auslösung der Injektionsvorrichtung in einem gespannten Zustand hält und die rotatorisch und axial in dem Gehäuse 270 bewegbar ist, wobei die Drehhülse 240 an ihrem äusseren Umfang mindestens eine Fassung 242, mindestens eine erste Führungsrippe 248b und an ihrem inneren Umfang mindestens eine Stützfläche 248a aufweist, eine Nadelschutzfeder 220, die die Energie für die Verschiebung der Nadelschutzeinrichtung 210 in die Nadelschutzposition oder eine zweite Position liefert, und eine Steuerhülse 300, welche axial beweglich und rotatorisch fest in dem Gehäuse angebracht wird und wobei die Steuerhülse 300 an ihrem Umfang mindestens eine Zange 300a aufweist, welche an ihrer Aussenseite mindestens eine Sperrrippe 301 und an ihrer Innenseite mindestens einen Zangennocken 302 aufweist.

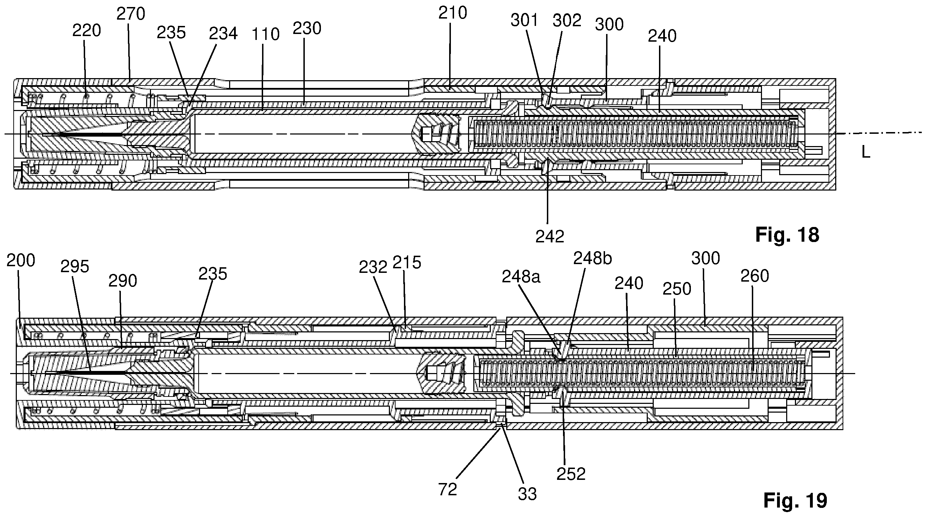

In den Figuren 18 und 19 wird die Injektionsvorrichtung in einem Auslieferungszustand gezeigt. An dem distalen Ende der Injektionsvorrichtung ist, wie im ersten Ausführungsbeispiel eine Kappe 200 angeordnet, die von dem Gehäuse 270 abziehbar ist und beim Abziehen in eine die Nadel 295 abdeckende Nadelschutzkappe 290 eingreift und diese bei der Abziehbewegung mitnimmt.

Das distale Ende der Nadelschutzeinrichtung 210 überragt das distale Ende des Gehäuses 270 der Injektionsvorrichtung. Das Antriebsmittel 260 ist zwischen Antriebseinrichtung 250 und Drehhülse 240 vorgespannt. Eine Verschiebung der Antriebseinrichtung 250 in Richtung Ausschüttposition wird durch den Eingriff der Stützfläche 248a an der Drehhülse 240 in die Halterungsfläche 252 der Antriebseinrichtung 250 verhindert. Die Stützfläche 248a wird durch die Innenseite der Steuerhülse 300 im Eingriff mit der Halterungsfläche 252 gehalten. Eine axiale Bewegung der Steuerhülse 300 wird verhindert, indem der Zangennocken 302 an der Steuerhülse 300 in die Fassung 242 an der Drehhülse 240 eingreift, dieser Eingriff wird über die Sperrrippe 301 an der Steuerhülse 300 und über die Innenfläche der Nadelschutzeinrichtung 210 gesichert.

Zwischen der Nadelschutzeinrichtung 210 und dem Haltering 235 ist die Nadelschutzfeder 220 vorgespannt. Die Nadelschutzeinrichtung 210 wird in ihrer distalen Ausgangsposition gegen ein Herausfallen aus der Injektionsvorrichtung mittels mindestens einem Anschlag 215 an der Nadelschutzeinrichtung 210 und mindestens einem Anschlag 232 an dem Spritzenhalter 230 gehalten.

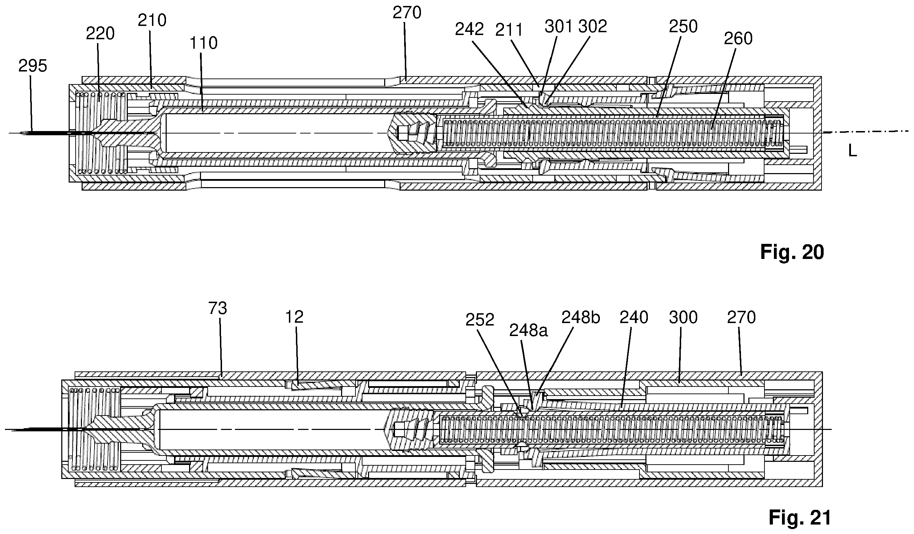

Die Figuren 20 und 21 zeigen die Injektionsvorrichtung in einem ausgelösten Zustand. Zum Auslösen der Injektionsvorrichtung wird zunächst die Kappe 200 und die Nadelschutzkappe 290 von der Injektionsvorrichtung entfernt. Danach umgreift der Anwender das Gehäuse 270 und drückt das distale Ende der Nadelschutzeinrichtung 210 gegen die Injektionsstelle. Hierdurch wird die Nadelschutzeinrichtung 210 relativ zum Gehäuse in proximale Richtung verschoben. Dadurch wird die Nadelschutzfeder 220 gespannt, d. h. die Nadelschutzeinrichtung 210 wird gegen die Kraft der Nadelschutzfeder 220 in proximale Richtung bewegt. Dadurch gelangt zumindest eine erste Nadelschutzeinrichtungsaussparung 211 an die Sperrrippe 301 der Steuerhülse 300, wodurch sich der Zangennocken 302 aus dem Eingriff mit der Fassung 242 quer zur Längsachse L lösen kann und die axiale Bewegung der Steuerhülse 300 freigegeben wird.

Über die axiale Bewegung der Nadelschutzeinrichtung 210 wird die Steuerhülse 300 in proximale Richtung verschoben, wodurch eine Bewegung quer zur Längsache L der ersten Führungsrippe 248b und der Stützfläche 248a freigegeben wird. Mittels der Kraft des Antriebsmittels 260 gelangt die Stützfläche 248a aus dem Eingriff mit der Halterungsfläche 252 und gibt die Antriebseinrichtung 250 in Richtung Ausschüttposition frei. Somit wird die Ausschüttsequenz gestartet.

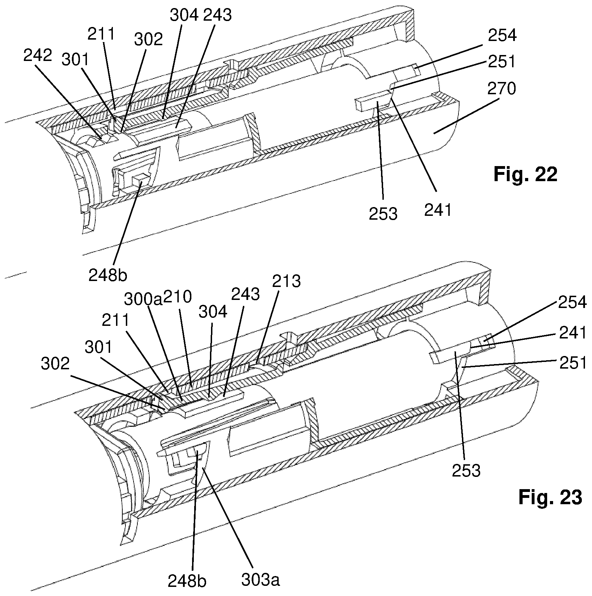

In der Figur 22 wird die Injektionsvorrichtung kurz nach dem Start der Ausschüttsequenz gezeigt, die Drehhülse 240 befindet sich in ihrer ersten Position. Durch die Kraft des Antriebsmittels 260 ist die Stützfläche 248a aus dem Eingriff mit der Halterungsfläche 252 gelangt. Mittels der Aussenwand des Antriebsmittels 250 wird die Zange 248 mit der Stützfläche 248a und der ersten Führungsrippe 248b quer zur Längsachse L nach aussen hin gespannt. Da das Antriebsmittel 260 zwischen der Antriebseinrichtung und der Drehhülse eingespannt ist, wird durch die Kraft des Antriebsmittels 260 die Drehhülse 240 in proximale Richtung gedrückt.

Mittels zumindest einem ersten Profil 241 an der Drehhülse 240 und zumindest einem zweiten Profil 251 am Gehäuse 270 oder an einem zusätzlichen Element am Gehäuse 270 wird ein Getriebe gebildet, welches durch die Kraft des Antriebsmittels 260, die Drehhülse 240 bis zu ihrer zweiten Position dreht, und der Nocken 253 in die Nut 254 des Gehäuses 270 gelangt. Gleichzeitig wird auch die quer zur Längsachse L nach aussen gespannte erste Führungsrippe 248b gedreht, wie auf der Figur 23 erkennbar ist.

Wie aus der Figur 23 ebenfalls ersichtlich ist, ist eine weitere proximale Bewegung der Drehhülse 240 über die nach aussen gespannte erste Führungsrippe 248b und der Haltefläche 303a an der Steuerhülse 300 verhindert. Des Weiteren gleitet über die Drehung der Drehhülse 240 mindestens eine nach innen zeigende Zangenabragung 304 an der Rampe 243 entlang und bewegt sich quer zur Längsachse L nach aussen hin, wodurch sich die Zange 300a ebenfalls quer zur Längsachse L nach aussen bewegt und gespannt wird und wodurch die Sperrrippe 301 eine Sperrposition für die Nadelschutzeinrichtung 210 einnimmt.

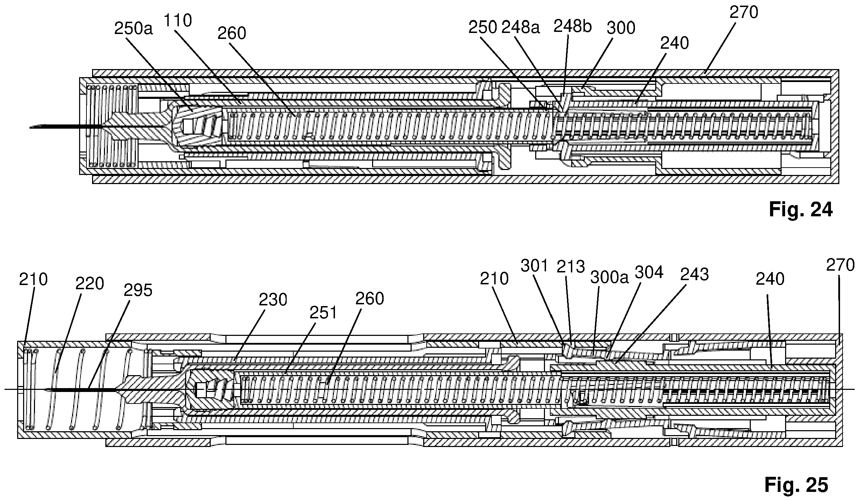

In der Figur 24 ist die Ausschüttsequenz beendet, die Injektionsvorrichtung befindet sich in einer Ausschüttposition. Der Kolben 250a ist am distalen Ende seines Wegs angekommen. Der Wirkstoff ist aus dem Wirkstoffbehälter 110 ausgeschüttet. Die Antriebseinrichtung 250 ist so bemessen, dass am Ende der Wirkstoffausschüttung das proximale Ende der Antriebseinrichtung 250 axial auf Höhe der Stützfläche 248a positioniert ist. Alternativ weist die Aussenwand der Antriebseinrichtung 250 eine Ausnehmung oder einen kleineren Durchmesser auf, welcher am Ende der Produktausschüttung axial auf Höhe der Stützfläche 248a positioniert ist.

Sobald das proximale Ende der Antriebseinrichtung 250 die Stützfläche 248a passiert hat, kann sich die nach aussen vorgespannte Zange 248 mit der Stützfläche 248a wieder entspannen und durch die federnde Anordnung radial nach innen schnappen. Die Bewegung der Stützfläche 248a bewirkt die Bewegung der ersten Führungsfläche 248b aus dem Eingriff mit der Haltefläche 303a der Steuerhülse 300. Hierdurch ist die Drehhülse 240 für eine Bewegung in proximale Richtung freigegeben. Aufgrund der in ihm gespeicherten Energie kann das Antriebsmittel 260 die Drehhülse 240 relativ zu der Steuerhülse 300 in proximale Richtung bewegen, wodurch der Nocken 253 in seine ihm zugeordnete Nut 254 einfährt, bis der Nocken 253 an der Drehhülse 240 an einem Ende der Nut 254 axial anschlägt (Figur 23 bis 25). Durch diesen Anschlag wird ein Klickgeräusch erzeugt, das dem Anwender der Vorrichtung akustisch oder/ und taktil mitteilt, dass der Wirkstoff vollständig ausgeschüttet ist.

Anschliessend zieht der Anwender die Vorrichtung von der Injektionsstelle ab, wodurch die Nadelschutzfeder 220 die Nadelschutzeinrichtung 210 relativ zu dem Gehäuse 270 in distale Richtung verschieben kann. Die Nadelschutzeinrichtung 210 wird so weit verschoben, bis sie ihre Nadelschutzposition eingenommen hat, d. h. über das distale Ende der Nadel 295 steht und die Nadel 295 somit vor einem Zugriff schützt (Figur 25).

Während der Verschiebung der Nadelschutzeinrichtung 210 in distale Richtung, bleibt die quer zur Längsachse L nach aussen gebogene Zange 300a über die Innenfläche der Nadelschutzeinrichtung und die Sperrrippe 301 radial nach innen zur Längsachse L hin gebogen. Sobald die Nadelschutzeinrichtung 210 sich in der Nadelschutzposition befindet, federt die Zange 300a aus ihrer gespannten Position heraus und die Sperrrippe 301 schnappt in die zweite Nadelschutzeinrichtungsaussparung 213 an der Nadelschutzeinrichtung 210 ein. Die Nadelschutzeinrichtung 210 kann somit nicht mehr in proximale Richtung verschoben werden. Alternativ wird ein erneutes Eindrücken der Nadelschutzeinrichtung verhindert, indem das proximale Ende der Nadelschutzeinrichtung 210 gegen die Sperrrippe 301 anschlagen kann.

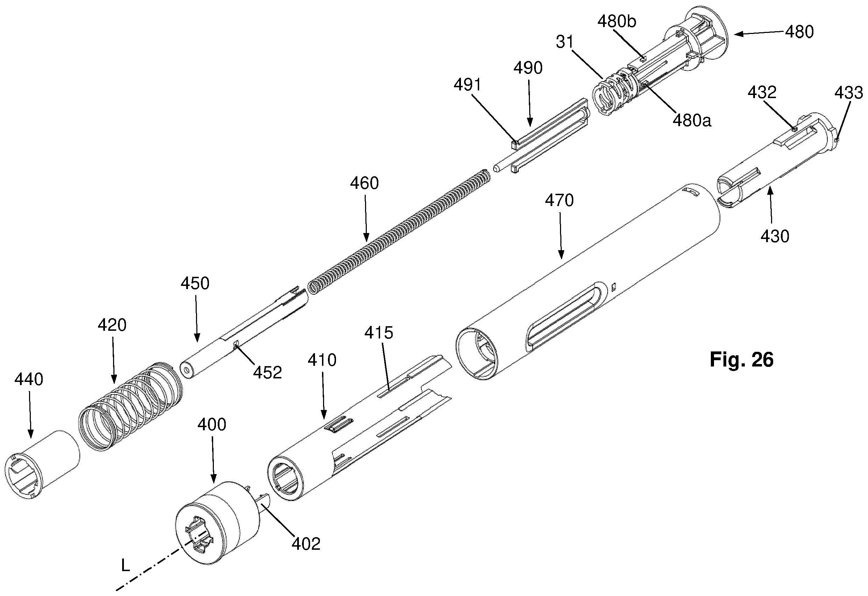

In den Figuren 26 bis 36 wird eine weitere vierte Ausführungsform einer Injektionsvorrichtung gezeigt.

In der Figur 26 werden die Einzelteile der Injektionsvorrichtung beschrieben. Die Injektionsvorrichtung umfasst eine Nadelschutzeinrichtung 410, ein hülsenförmiges Gehäuse 470, wobei die Nadelschutzeinrichtung 410 in dem Gehäuse 470 um die Längsachse L längsverschiebbar aufgenommen ist und gleichzeitig als Auslöseelement dient, ein Spritzenhalter 430 welcher axial fest mit dem Gehäuse 470 verbunden wird und worin ein Wirkstoffbehälter 110 eingesetzt werden kann, ein Haltering 435, welcher im Gehäuse 470 integriert sein kann um insbesondere den Wirkstoffbehälter 110 im Spritzenhalter 230 zu sichern, eine Antriebseinrichtung 450 welche auf den Wirkstoffbehälter 110 wirken kann, in der Gestalt einer Hülse welche an ihrem Umfang mindestens eine Halterungsfläche 452 aufweist und ein Antriebsmittel 460 in der Gestalt einer als Druckfeder wirkenden Wendelfeder, die die Energie für eine Ausschüttsequenz liefert, eine Drehhülse 440, die die Antriebseinrichtung 450 und das Antriebsmittel 460 bis zur Auslösung der Injektionsvorrichtung in einem gespannten Zustand hält und die rotatorisch und axial in dem Gehäuse 470 bewegbar ist, wobei die Drehhülse 440 an ihrem inneren Umfang Führungsbahnen aufweist welche zumindest aus einem ersten Profil 441a, aus einem vierten Profil 441b, aus einer ersten Führung 442, einer zweiten Führung 443 und einer Verriegelungsbahn 444 zusammengesetzt werden, eine Nadelschutzfeder 420, die die Energie für die Verschiebung der Nadelschutzeinrichtung 410 und der Drehhülse 440 in die Nadelschutzposition oder eine zweite Position liefert, eine Steuerhülse 480, welche axial fest in dem Gehäuse 470 angebracht ist und wobei die Steuerhülse 480 an ihrer Aussenwand mindestens ein zweites Profil 480a und ein drittes Profil 480b und an ihrer Innenwand mindestens einen inneren Auslöseschnapper 480c aufweist, eine Ausgleichsfeder 31, welche sich an dem proximalen Ende des Wirkstoffbehälters 110 abstützt und über die der Wirkstoffbehälter 110 in und gegen die Ausschüttrichtung elastisch am Gehäuse 470 abgestützt wird, und einen Endklicker 490 der am Ende der Ausschüttsequenz ein akustisches und/ oder taktiles Signal erzeugt.

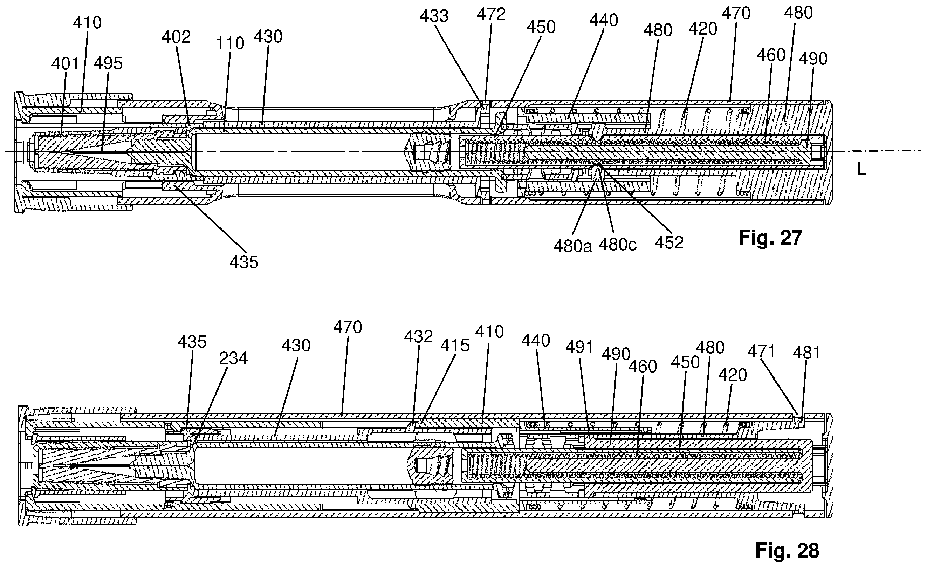

In den Figuren 27 und 28 wird die Injektionsvorrichtung in einem Auslieferungszustand gezeigt. An dem distalen Ende der Injektionsvorrichtung ist, wie in den vorhergehenden Ausführungsbeispielen eine Kappe 400 angeordnet, die von dem Gehäuse 470 abziehbar ist und beim Abziehen mit mindestens einer Klaue 402 in eine die Nadel 495 abdeckende Nadelschutzkappe 401 eingreift und diese bei der Abziehbewegung mitnimmt. Über den im Gehäuse 470 integrierten Haltering 435 werden die Klauen 402 der Kappe 400 radial nach innen zur Längsachse L hin insbesondere gehalten, wodurch die Klaue 402 durch Kraft- und Formschluss in die Nadelschutzkappe 401 eingreifen kann und diese beim Abziehen der Kappe 400 mitnimmt.

Das distale Ende der Nadelschutzeinrichtung 410 steht in distaler Richtung über das distale Ende des Gehäuses 470 der Injektionsvorrichtung vor. Das Antriebsmittel 460 ist zwischen der Antriebseinrichtung 450 und dem Endklicker 490 vorgespannt montiert. Der Endklicker 490 wird gegen ein proximales Verschieben mittels Armnocken 491 am distalen Ende oder an Halteschlitzen in der Steuerhülse 480 gehalten und die federnd vorgespannten Armnocken 491 werden mittels der Antriebseinrichtung 450 gegen eine radiale Bewegung nach innen gesichert. Eine Verschiebung der Antriebseinrichtung 450 in Richtung Ausschüttposition wird durch den Eingriff mindestens eines inneren Auslöseschnappers 480c an der Steuerhülse 480 in die Halterung 452 der Antriebseinrichtung 450 verhindert. Der innere Auslöseschnapper 480c wird durch die Innenwand der Steuerhülse 480 im Eingriff mit der Halterungsfläche 452 gehalten. Eine axiale Bewegung der Steuerhülse 480 gegenüber dem Gehäuse 470 wird verhindert, indem mindestens ein Nocken 481 an der Steuerhülse 480 in mindestens einen Halterungsschlitz 471 im Gehäuse 470 eingreift, insbesondere verschnappt.

Das distale Ende der Drehhülse 440 und das proximale Ende der Nadelschutzeinrichtung 410 liegen axial aufeinander auf, sind aber nicht rotatorisch gekoppelt. Alternativ können die Drehhülse 440 und die Nadelschutzeinrichtung 410 ein Bauteil sein, welches rotatorisch in sich beweglich ausgebildet ist. Die Nadelschutzfeder 420 ist zwischen der Drehhülse 440 und der Steuerhülse 480 vorgespannt. Die Nadelschutzeinrichtung 410 wird in ihrer distalen Ausgangsposition gegen ein Herausfallen aus der Injektionsvorrichtung mittels mindestens einem Anschlag 415 an der Nadelschutzeinrichtung 410 und mindestens einem Anschlag 432 an dem Spritzenhalter 430 gehalten.

Der Wirkstoffbehälter 110 ist in dem Spritzenhalter 430 angeordnet und stützt sich mit einer Schulter, die distal am Wirkstoffbehälter 110 angeordnet ist, an einer nach innen gerichteten Schulter 234 des Spritzenhalters 430 ab. Der Spritzenhalter 430 ist über zumindest ein Haltemittel 433 im Gehäusedurchbruch 472 axial fest mit dem Gehäuse 470 verbunden, insbesondere verschnappt. Der Spritzenhalter 430 ist an seinem distalen Ende im Bereich der Schulter 234 vom Haltering 435 im Gehäuse 470 umfasst, wodurch der Wirkstoffbehälter 110 mittels des Halterings 435 gesichert wird.

Ein bestimmter Anfangswiderstand sorgt dafür, dass die Anpresskraft des Anwenders einen bestimmten Wert übersteigen muss und die Nadel 495 dadurch mit genügend Kraft eingestochen wird. Hierzu kann entweder der Spritzenhalter 430 oder die Nadelschutzeinrichtung 410 mindestens einen flexiblen Schnapper aufweisen. Befinden sich die Schnapper am Spritzenhalter 430, können diese insbesondere radial nach aussen zur Nadelschutzeinrichtungsinnenfläche (nicht gezeigt) ausgerichtet sein, wobei die Nadelschutzeinrichtung 410 auf der Innenseite mindestens ein Sperrmittel (nicht gezeigt) aufweist. Befinden sich die Schnapper an der Nadelschutzeinrichtung 410, können diese insbesondere nach innen zur Spritzenhalteraussenfläche ausgebildet sein, wobei der Spritzenhalter 430 auf der Aussenseite mindestens ein Sperrmittel aufweist. Wird bei dem Versuch die Nadelschutzeinrichtung 410 in das Gehäuse 470 zurückzuschieben der Schnapper gegen das Sperrmittel gedrückt, kann der Schnapper zunächst nicht den Widerstand des Sperrmittels überwinden. Dieser Anfangswiderstand behindert das Zurückschieben der Nadelschutzeinrichtung 410, so dass beim Andrücken der Injektionsvorrichtung an die Injektionsstelle zunächst die bestimmte Anpresskraft zur Überwindung des Anfangswiderstands aufgewendet werden muss, die sobald sie den Schnapper aus dem Eingriff mit dem Sperrmittel gedrückt hat, schlagartig wegfällt, wodurch die Nadelschutzeinrichtung 410 schlagartig zurückgeschoben und die Nadel 495 schlagartig eingestochen wird.

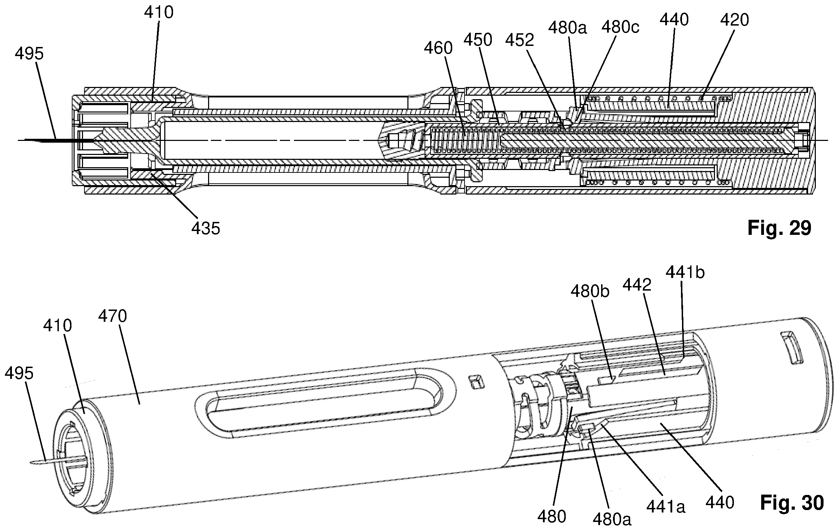

Die Figuren 29 und 30 zeigen die Injektionsvorrichtung in einem ausgelösten Zustand. Zum Auslösen der Injektionsvorrichtung wird zunächst die Kappe 400 und die Nadelschutzkappe 401 durch Abziehen von der Injektionsvorrichtung entfernt. Danach umgreift der Anwender das Gehäuse 470 und drückt das distale Ende der Nadelschutzeinrichtung 410 gegen die Injektionsstelle. Hierdurch wird die Nadelschutzeinrichtung 410 zusammen mit der Drehhülse 440 relativ zum Gehäuse in proximale Richtung verschoben. Dadurch wird die Nadelschutzfeder 420 gespannt, d. h. die Nadelschutzeinrichtung 410 wird gegen die Kraft der Nadelschutzfeder 420 in proximale Richtung bewegt.

Über die axiale Bewegung der Nadelschutzeinrichtung 410 wird die Drehhülse 440 in proximale Richtung verschoben, wodurch die Innenwand der Steuerhülse 480 und das erste Profil 441a an der Drehhülse 440 an dem inneren Auslöseschnapper 480c an der Steuerhülse 480 vorbeibewegt werden und wodurch die Bewegung der inneren Auslöseschnapper 480c nach aussen quer zur Längsache L freigegeben wird.

Des Weiteren wird über die axiale Bewegung der Steuerhülse 480 mindestens ein drittes Profil 480b an der Steuerhülse 480 von einer proximalen Position, aus einer ersten Führung 442 in eine distale Position hinaus bewegt, wodurch die Drehung der Drehhülse 440 freigegeben wird. Mittels der Kraft des Antriebsmittels 460 gelangt der innere Auslöseschnapper 480c aus dem Eingriff mit der Halterungsfläche 452 und gibt die Antriebseinrichtung 450 in Richtung Ausschüttposition frei. Somit wird die Ausschüttsequenz gestartet, das Antriebsmittel 460 treibt die Antriebseinrichtung 450 in Richtung Ausschüttposition, die Antriebseinrichtung bewegt den Kolben 450a in distale Richtung und der Wirkstoff wird aus dem Wirkstoffbehälter 110 über die Nadel 495 ausgeschüttet.

Die Figur 30 zeigt die Injektionsvorrichtung kurz nach dem Start der Ausschüttsequenz, wobei sich die Drehhülse 440 in ihrer ersten Position befindet. Durch die Kraft des Antriebsmittels 460 sind die inneren Auslöseschnapper 480c aus dem Eingriff mit der Halterungsfläche 452 gelangt. Auf dem äusseren Umfang der Steuerhülse 480 befindet sich an derselben Zange wie die inneren Auslöseschnapper 480c zumindest ein zweites Profil 480a. Mittels der Aussenwand der Antriebseinrichtung 450 werden die inneren Auslöseschnapper 480c und das zweite Profil 480a quer zur Längsachse L nach aussen gespannt. Dabei liegen das erste Profil 441a an der Drehhülse 440 und das zweite Profil 480a an der Steuerhülse 480 unmittelbar aufeinander auf und können ein Getriebe bilden.

Bevorzugt wird über das quer zur Längsachse L nach aussen gespannte zweite Profil 480a des so gebildeten Getriebes die Drehung der Drehhülse 440 in ihre Sperrposition ermöglicht.

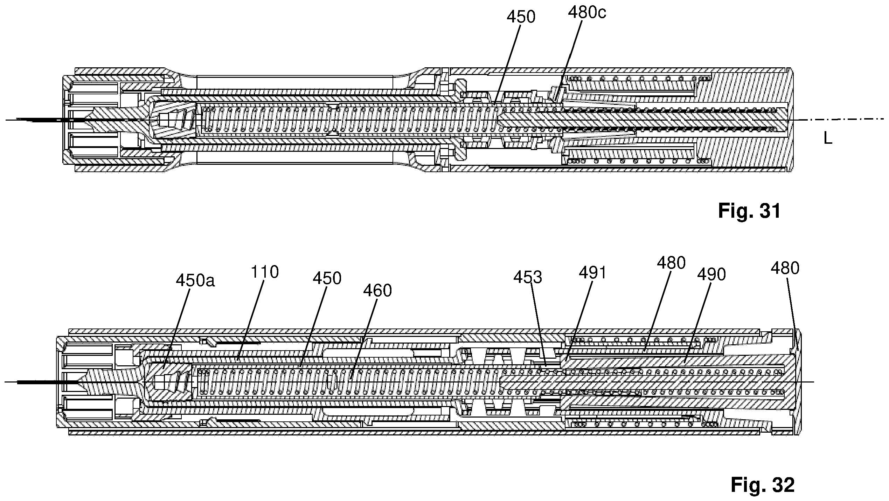

In den Figuren 31 und 32 ist die Ausschüttsequenz beendet. Der Kolben 450a ist am distalen Ende seines Wegs angekommen. Der Wirkstoff ist aus dem Wirkstoffbehälter 110 ausgeschüttet. Die Antriebseinrichtung 450 ist so bemessen, dass am Ende der Wirkstoffausschüttung, axial auf Höhe der Armnocken 491 des Endklickers 490 eine Ausnehmung 453 positioniert ist. Alternativ weist die Antriebseinrichtung 450 einen kleineren Durchmesser auf oder das proximale Ende der Antriebseinrichtung 450 ist am Ende der Produktausschüttung axial auf der Höhe des Armnockens 491 positioniert.

Sobald das proximale Ende der Antriebseinrichtung 450 den Armnocken 491 passiert hat, kann sich der Armnocken 491 über seine vorgespannte federnde Anordnung radial nach innen bewegen. Die Bewegung der Armnocken 491 radial nach innen, geben die axiale Bewegung des Endklickers 490 in proximale Richtung frei. Aufgrund der in ihm gespeicherten Energie kann das Antriebsmittel 460 den Endklicker 490 relativ zu der Steuerhülse 480 und zum Gehäuse 470 in proximale Richtung bewegen, bis der Endklicker 490 axial am Gehäuse 470 oder an der Steuerhülse 480 anschlägt (Figur 32). Durch diesen Anschlag wird ein Klickgeräusch erzeugt, das dem Anwender der Vorrichtung akustisch oder/ und taktil mitteilt, dass der Wirkstoff vollständig ausgeschüttet ist.

Anschliessend zieht der Anwender die Vorrichtung von der Injektionsstelle ab, wodurch die Nadelschutzfeder 420 die Nadelschutzeinrichtung 410 relativ zu dem Gehäuse 470 in distale Richtung verschieben kann. Die Nadelschutzeinrichtung 410 wird so weit verschoben, bis sie ihre Nadelschutzposition einnimmt, d. h. über das distale Ende der Nadel 495 steht und die Nadel 495 somit vor einem Zugriff schützt.

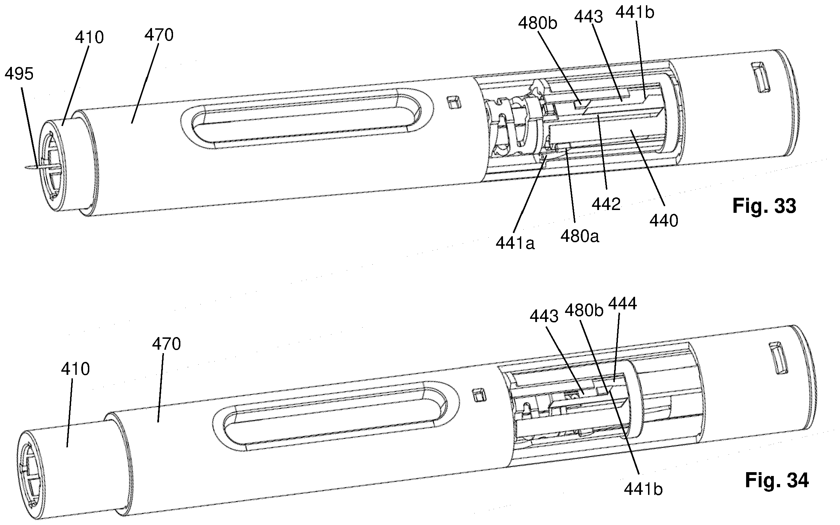

Durch die Kraft der Nadelschutzfeder 420 bewegen sich die Nadelschutzeinrichtung 410 und die Drehhülse in distale Richtung. Über das erste Profil 441a an der Drehhülse 440 und das zweite Profil 480a an der Steuerhülse 480 wird das Getriebe gebildet, welches über die Kraft der Nadelschutzfeder 420 die Drehhülse 440 aus einer Start-Position soweit dreht, dass das dritte Profil 480b an der Steuerhülse 480 vor der zweiten Führung 443 an der Drehhülse 440 steht. Wodurch nun die axiale Bewegung der Nadelschutzeinrichtung 410 in ihre Nadelschutzposition weiter voranschreiten kann und wodurch das dritte Profil 480b durch die distale Bewegung der Nadelschutzeinrichtung durch die zweite Führung 443 gleiten kann. (Figur 33).

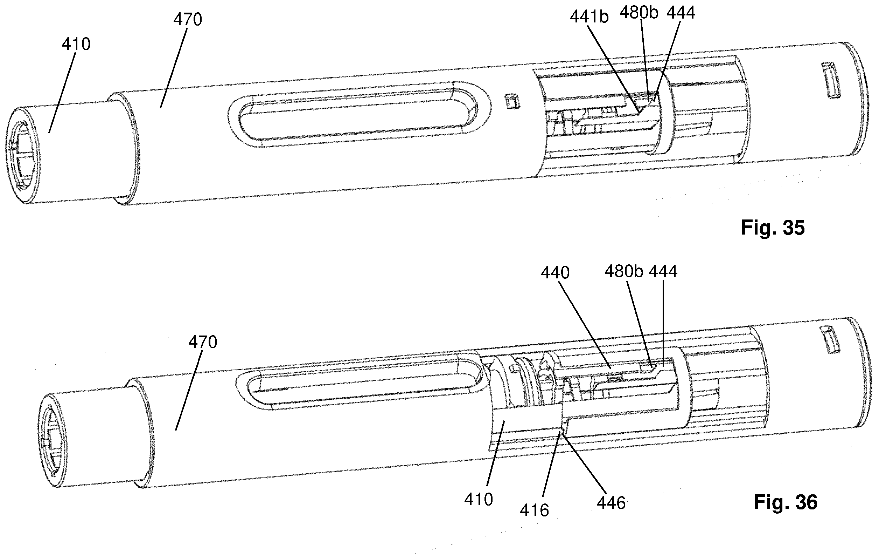

Dadurch bewegt sich die Nadelschutzeinrichtung 410 weiter in distale Richtung in ihre Nadelschutzposition, wobei sich das dritte Profil 480b am proximalen Ende der zweiten Führung 443 befindet. Am proximalen Ende der zweiten Führung ist ein viertes Profil 441b angebracht. Dieses bildet mit dem dritten Profil 480b ein Getriebe wodurch die Drehhülse 440 durch die Kraft der Nadelschutzfeder 420 erneut gedreht wird und das dritte Profil 480b in die Verriegelungsbahn 444 gelangt (Figur 35).

Über diese Drehung gelangt die Drehhülse 440 in eine Sperrposition. Weiterhin bewegt sich der Zentriernocken 416 an der Nadelschutzeinrichtung 410 in eine Zentriernut 446 an der Drehhülse, wodurch ein erneutes Drehen der Drehhülse 440 verhindert wird. Ausserdem kann die Nadelschutzeinrichtung 410 nicht mehr in proximale Richtung verschoben werden und befindet sich in ihrer gesperrten Nadelschutzposition, da das dritte Profil 480b an der Steuerhülse 480 in der Verriegelungsbahn 444 ein erneutes Eindrücken der Nadelschutzeinrichtung 410 verhindert (Figur 36).

In einer alternativ bevorzugen Ausführung, kann die Drehung der Drehhülse 440 aus der Start-Position in die Sperrposition über ein einziges Profil an der Steuerhülse 480 erfolgen. So kann z. B. über das zweite Profil 480a an der Steuerhülse die Drehhülse aus der Start-Position soweit gedreht werden, dass die axiale Bewegung der Drehhülse und der Nadelschutzeinrichtung 410 in ihre Nadelschutzposition weiter voranschreiten kann. Dabei kann am proximalen Ende der Drehhülse 440 ein weiteres Profil angebracht sein und mittels demselben zweiten Profil 480a ein Getriebe bilden, wodurch die Drehhülse 440 durch die Kraft der Nadelschutzfeder 420 erneut gedreht wird und in die Sperrposition gelangt.

### Bezugszeichen:

10, 210, 410 Nadelschutzeinrichtung
11, 111 Schnappelement
12 Schnapper
14, 114, 301 Sperrrippe
15, 215, 415 Anschlag
20, 220, 420 Nadelschutzfeder
30, 230, 430 Spritzenhalter
31 Ausgleichsfedern
32, 232, 432 Anschlag
33, 433 Haltemittel
34, 234 Schulter
235, 435 Haltering
40, 140, 240, 440 Drehhülse
40a Anfangsanschlag
40b, 140b erste Führung
40c zweite Führung /Rückdrehsperre
41, 241, 441a erstes Profil
42, 248b erste Führungsrippe
43 erste Nut
43a zweite Nut
44 zweite Führungsrippe
45 Rampe
45a Endanschlag
46 Klaue
47, 248a Stützfläche
48 Führung
49 Nocken
50, 250, 450 Antriebseinrichtung
250a, 450a Kolben
51, 251, 480a zweites Profil
52 Halterung
53 Klickelement,
253 Nocken
54, 252, 452 Halterungsfläche
55 Aussparung
60, 260, 460 Antriebsmittel
70, 270, 470 Gehäuse
71, 471 Halterungsschlitz
72, 472 Gehäusedurchbruch
73 Sperrmittel
80 Knopf
81 Gegenklaue
82 Drehknopf
90 Kappeneinsatz
95, 295, 495 Nadel
100, 200, 400 Kappe
101, 290, 401 Nadelschutzkappe
102 Kolben
110 Wirkstoffbehälter
140a Getriebefläche
211 erste Nadelschutzeinrichtungsaussparung
213 zweite Nadelschutzeinrichtungsaussparung
242 Fassung
243 Rampe
248 Zange
254 Nut
300, 480 Steuerhülse
300a Zange
302 Zangennocken
303a Haltefläche
304 Zangenabragung
402 Klaue
416 Zentriernocken
441b viertes Profil
442 erste Führung
443 zweite Führung
444 Verriegelungsbahn
446 Zentriernut
453 Ausnehmung
480b drittes Profil
480c innere Auslöseschnapper
481 Nocken
490 Endklicker
491 Armnocken
L Längsachse

## Patentansprüche

1. Injektionsvorrichtung zum automatischen Ausschütten umfassend:
a) ein Gehäuse (470) mit einer Längsachse (L),
b) eine Nadelschutzeinrichtung (410) die entlang der Längsachse (L) von einer distalen Ausgangsposition in eine proximale Position und aus der proximalen Position in eine Nadelschutzposition verschiebbar ist,
c) eine im Gehäuse (470) bewegbare Antriebseinrichtung (450), welche durch ein Antriebsmittel (460) in eine Ausschüttposition getrieben wird,
d) eine Steuerhülse (480), welche axial fest im Gehäuse (470) angebracht ist,
e) ein Spritzenhalter (430) aufweisend eine nach innen gerichtete Schulter (234), an welcher sich ein in dem Spritzenhalter angeordneter Wirkstoffbehälter (110) mit einer distal angeordneten Schulter abstützt,
**dadurch gekennzeichnet, dass** die Injektionsvorrichtung aufweist
f) einen im Gehäuse (470) integrierten Haltering (435) zur Sicherung des Wirkstoffbehälters (110) im Spritzenhalter (430), wobei der Haltering (435) den Spritzenhalter (430) im Bereich der Schulter (234) umfasst, und
g) einen Endklicker (490) welcher im Auslieferungszustand gegen ein proximales Verschieben aufgrund der gespeicherten Energie des Antriebsmittels (460) mittels radial vorgespannt angeordneter Armnocken (491) des Endklickers (490) in der Steuerhülse (480) gehalten ist, wobei am Ende der Wirkstoffausschüttung die Armnocken (491) sich radial nach innen bewegen und eine proximale Bewegung des Endklickers (490) bis zu einem Anschlag an Gehäuse (470) oder Steuerhülse (480) freigeben.

2. Injektionsvorrichtung nach Anspruch 1, **dadurch gekennzeichnet, dass** im Auslieferungszustand die radial vorgespannt angeordneten Armnocken (491) durch die Antriebseinrichtung (450) gegen eine radiale Bewegung nach innen gesichert sind, und dass am Ende der Wirkstoffausschüttung axial auf der Höhe der Armnocken (491) eine Ausnehmung (453), ein kleinerer Durchmesser, oder ein proximales Ende der Antriebseinrichtung (450) positioniert ist.

3. Injektionsvorrichtung nach Anspruch 1, **gekennzeichnet durch** eine Ausgleichsfeder (31), welche sich an einem proximalen Ende des Wirkstoffbehälters 110 abstützt, und über die der Wirkstoffbehälter (110) elastisch am Gehäuse (470) abgestützt wird.

4. Injektionsvorrichtung nach Anspruch 1, **dadurch gekennzeichnet, dass** die Steuerhülse (480) einen inneren Auslöseschnapper (480c) aufweist, welcher im Auslieferungszustand in eine Halterung (452) der Antriebseinrichtung (450) eingreift und dadurch eine Verschiebung der Antriebseinrichtung (450) in Richtung Ausschüttposition verhindert.

5. Injektionsvorrichtung nach Anspruch 4, **dadurch gekennzeichnet, dass** der Eingriff gesichert ist durch eine Drehhülse (440), welche durch eine axiale Bewegung der Nadelschutzeinrichtung (410) verschoben wird, wodurch die Bewegung des inneren Auslöseschnappers (480c) nach aussen quer zur Längssachse L freigegeben wird.

6. Injektionsvorrichtung nach Anspruch 1, **dadurch gekennzeichnet, dass** ein Nocken (481) der Steuerhülse (480) in einem Halterungsschlitz (471) im Gehäuse (470) verschnappt ist.

## Claims

1. Injection device for automatic dispensing comprising:
a) a housing (470) having a longitudinal axis (L),
b) a needle protection mechanism (410) which is displaceable along the longitudinal axis (L) from a distal starting position to a proximal position and from the proximal position to a needle protection position,
c) a drive mechanism (450) which is movable in the housing (470) and which is driven into a dispensing position by a drive means (460),
d) a control sleeve (480) which is axially rigidly mounted in the housing (470),
e) a syringe holder (430) having an inwardly directed shoulder (234) on which an active-substance container (110) arranged in the syringe holder is supported via a distally arranged shoulder, **characterized in that** the injection device has
f) a retaining ring (435) integrated in the housing (470) for securing the active-substance container (110) in the syringe holder (430), the retaining ring (435) enclosing the syringe holder (430) in the region of the shoulder (234), and
g) an end clicker (490) which, in the delivery state, is held in the control sleeve (480) against proximal displacement due to the stored energy of the drive means (460) by means of radially prestressed arm cams (491) of the end clicker (490), the arm cams (491), at the end of the active substance dispensation, moving radially inward and releasing a proximal movement of the end clicker (490) up to a stop on the housing (470) or control sleeve (480).

2. Injection device according to claim 1, **characterized in that** in the delivery state, the radially prestressed arm cams (491) are secured against radial inward movement by the drive mechanism (450), and **in that** at the end of the active substance dispensation, a recess (453), a smaller diameter, or a proximal end of the drive mechanism (450) is positioned axially at the height of the arm cams (491).

3. Injection device according to claim 1, **characterized by** a compensating spring (31) which is supported on a proximal end of the active substance container 110 and via which the active substance container (110) is resiliently supported on the housing (470).

4. Injection device according to claim 1, **characterized in that** the control sleeve (480) has an inner release catch (480c) which, in the delivery state, engages in a holder (452) of the drive mechanism (450) and thereby prevents a displacement of the drive mechanism (450) in the direction of the dispensing position.

5. Injection device according to claim 4, **characterized in that** the engagement is secured by a rotating sleeve (440), which is displaced by an axial movement of the needle protection mechanism (410), as a result of which the movement of the inner release catch (480c) toward the outside transversely to the longitudinal axis L is released.

6. Injection device according to claim 1, **characterized in that** a cam (481) of the control sleeve (480) is snapped into a retaining slot (471) in the housing (470).

## Revendications

1. Dispositif d'injection destiné au déversement automatique, comprenant :
a) un boîtier (470) comportant un axe longitudinal (L),
b) un appareil de protection d'aiguille (410) qui peut être coulissé le long de l'axe longitudinal (L) d'une position initiale distale à une position proximale et de la position proximale à une position de protection d'aiguille,
c) un appareil d'entraînement (450) pouvant se déplacer dans le boîtier (470), lequel appareil d'entraînement est entraîné dans une position de déversement par un moyen d'entraînement (460),
d) une douille de commande (480) qui est montée axialement de manière fixe dans le boîtier (470),
e) un élément de retenue de seringue (430) présentant un épaulement (234) orienté vers l'intérieur sur lequel s'appuie un récipient de substance active (110) comportant un épaulement disposé de manière distale, lequel récipient de substance active est disposé dans l'élément de retenue de seringue,
**caractérisé en ce que** le dispositif d'injection présente
f) une bague de retenue (435) intégrée dans le boîtier (470) et permettant le blocage du récipient de substance active (110) dans l'élément de retenue de seringue (430), dans lequel la bague de retenue (435) comprend l'élément de retenue de seringue (430) dans la zone de l'épaulement (234), et
g) un cliquet de fin (490) qui, à l'état de distribution, est retenu dans la douille de commande (480) contre un coulissement proximal en raison de l'énergie accumulée du moyen d'entraînement (460) au moyen de cames formant bras (491) du cliquet de fin de déversement de substance (490) disposées de manière précontrainte radialement, dans lequel les cames formant bras (491) se déplacent radialement vers l'intérieur à la fin du déversement de substance active et déclenchent un déplacement proximal du cliquet de fin (490) jusqu'à une butée au niveau du boîtier (470) ou de la douille de commande (480).

2. Dispositif d'injection selon la revendication 1, **caractérisé en ce que,** à l'état de distribution, les cames formant bras (491) disposées de manière précontrainte radialement sont bloquées à l'encontre d'un déplacement radial vers l'intérieur par l'appareil d'entraînement (450), **et en ce qu'**un évidement (453), un diamètre plus petit, ou une extrémité proximale de l'appareil d'entraînement (450) est positionné axialement par rapport à la hauteur des cames formant bras (491) à la fin du déversement de substance active.

3. Dispositif d'injection selon la revendication 1, **caractérisé par** un ressort d'équilibrage (31) qui s'appuie sur une extrémité proximale du récipient de substance active (110) et par l'intermédiaire duquel le récipient de substance active (110) s'appuie élastiquement sur le boîtier (470).

4. Dispositif d'injection selon la revendication 1, **caractérisé en ce que** la douille de commande (480) présente un déclic de libération interne (480c) qui, à l'état de distribution, est mis en prise dans un élément de retenue (452) de l'appareil d'entraînement (450) et empêche ainsi un coulissement de l'appareil d'entraînement (450) en direction de la position de déversement.

5. Dispositif d'injection selon la revendication 4, **caractérisé en ce que** la mise en prise est assurée par un manchon rotatif (440) qui est coulissé par un déplacement axial de l'appareil de protection d'aiguille (410), moyennant quoi le déplacement du déclic de libération interne (480c) vers l'extérieur transversalement par rapport à l'axe longitudinal (L) est déclenché.

6. Dispositif d'injection selon la revendication 1, **caractérisé en ce qu'une** came (481) de la douille de commande (480) est encliquetée dans une fente de retenue (471) dans le boîtier (470).
